Europäisches Patentamt

**European Patent Office**

Office européen des brevets

(19)

(11) Veröffentlichungsnummer: **0 028 364**
**B1**

(12)

# EUROPÄISCHE PATENTSCHRIFT

(45) Veröffentlichungstag der Patentschrift:
**20.06.84**

(51) Int. Cl.³: **A 61 M 5/14,** G 05 D 7/06

(21) Anmeldenummer: **80106462.7**

(22) Anmeldetag: **23.10.80**

(54) **Vorrichtung zum Infundieren von Flüssigkeiten.**

(30) Priorität: **02.11.79 DE 2944186**

(43) Veröffentlichungstag der Anmeldung:
**13.05.81 Patentblatt 81/19**

(45) Bekanntmachung des Hinweises auf die Patenterteilung:
**20.06.84 Patentblatt 84/25**

(84) Benannte Vertragsstaaten:
**AT BE CH DE FR GB IT LI LU NL SE**

(56) Entgegenhaltungen:
**DE - A - 2 529 636**
**GB - A - 2 000 833**
**US - A - 3 736 930**

**CONTROL ENGINEERING, Band 23, Nr. 1, Januar 1976, South Riverside Playa Chicago, US WILSON: "Weight-feeder control needs favor the digital route", Seiten 48, 49**

(73) Patentinhaber: **Labionics Aktiengesellschaft, Ziegelbrückenstrasse 22a, CH-8867 Niederurnen (CH)**

(72) Erfinder: **Hagen, Claus-Dieter, Kuehnstrasse 6, D-2000 Hamburg 70 (DE)**

## Beschreibung

Die Erfindung bezieht sich auf eine Vorrichtung zum Infundieren von Flüssigkeiten, mit einer Pumpe in einem Schlauchsystem zwischen einem Vorrat an Infusionslösung und einer Infusionskanüle oder dergleichen, einem in seiner Drehzahl steuerbaren Antriebmotor für die Pumpe, einer ein elektronisches Rechnerbauteil enthaltenden Steuereinrichtung für den Motor, die eine Wähleinrichtung für den SOLL-Wert der von der Pumpe zu fördernden Flüssigkeitsmenge umfasst, mit einer Fühlereinrichtung, die die Förderleistung der Pumpe misst und ein IST-Signal über einen Steuereingang auf das elektronische Rechnerbauteil gibt, das abhängig von einem SOLL-IST-Wertvergleich das Steuersignal für den Motor bildet, sowie mit einer an das Rechnerbauteil angeschlossenen Alarmeinrichtung für einen vorgegebenen Grenzwert des SOLL-IST-Wertvergleichs.

Bei Geräten zum Infundieren und Dosieren von Flüssigkeiten unterscheidet man grundsätzlich zwischen Infusionsregler durch Tropfenzählung und motorisch getriebenen Infusionspumpen unterschiedlicher Bauart. Der Infusionsregler steuert die sogenannte Schwerkraftinfusion, indem eine Ventilvorrichtung am Infusionsschlauch eine vorgegebene Tropfenrate zulässt. Es ist bekannt, die Ventilvorrichtung elektronisch zu steuern. Ein Tropfensensor ermittelt die tatsächlich verabreichte Tropfenrate, und eine Überwachungseinheit vergleicht die tatsächliche Tropfenrate mit einem Sollwert. Die Tropfenzahl gibt jedoch nur einen ungefähren Anhalt über die tatsächlich infundierte Flüssigkeitsmenge, da eine Reihe von Faktoren die Beziehung von Tropfenzahl zur Flüssigkeitsmenge beeinflusst. Genannt seien etwa die Viskosität und die Temperatur der Flüssigkeit oder auch die Form des Einlaufstutzens in die Tropfenzählkammer. Die Tropfengrösse kann um 30% und mehr variieren.

Mit Hilfe von motorgetriebenen Infusionspumpen lässt sich die Flüssigkeitsfördermenge verhältnismässig genau einstellen. Infusionspumpen können beispielsweise Kolbenpumpen, Flügelzellpumpen und vor allen Dingen Schlauchpumpen sein. Ihr Antrieb erfolgt normalerweise mit Hilfe eines sogenannten Schrittmotors, der sich bekanntlich verhältnismässig genau in seiner Drehzahl steuern lässt, so dass über die volumetrischen Pumpen auch die Förderleistung in einem vorgegebenen Bereich verhältnismässig genau eingestellt werden kann. Eine von einem Schrittmotor angetriebene Infusionspumpe ist etwa aus der DE-A-2 529 636 bekannt.

Es hat sich gezeigt, dass auch volumetrische Infusionspumpen Schwankungen in der Fördermenge pro Zeiteinheit unterliegen können, vor allen Dingen bei grösseren Infusionszeiträumen.

Aus der US-A-3 736 930 ist ein Regelungssystem für die Fördermenge einer Infusionspumpe bekannt, bei dem die Impulsraten eines von einem Tropfensensor gesteuerten Generators und eines einstellbaren Sollwertgenerators verglichen werden und das dabei erhaltene Differenzsignal einem variablen Impulsgenerator zugeführt wird, dessen Ausgangsimpulse mittels einer Motorsteuerung den Schrittmotor der Infusionspumpe schalten. Dabei dient eine Überwachungsschleife, bestehend aus einem von einem Rotationssensor angesteuerten Zähler, dessen Rücksetzeingang mit dem Tropfensensor verbunden ist, zur Verhinderung des Pumpenleerlaufs und damit des Einziehens von Luft bei der Infusion, so dass nach Ausbleiben der Tropfensensorimpulse während einer bestimmten Anzahl von Schrittmotorumdrehungen ein Alarmsystem aktiviert wird. Für die Fördermengenregelung ist ebenfalls eine Alarmierung vorgesehen, bei der das aus dem SOLL-IST-Wertvergleich erhaltene Differenzsignal überwacht wird. Eine Unsicherheit besteht darin, dass sich die Regelung und Überwachung der Fördermenge auf nur eine IST-Wertermittlung beziehen.

Vorliegender Erfindung liegt dementsprechend die Aufgabe zugrunde, eine Vorrichtung zum Infundieren von Flüssigkeiten zu entwickeln, bei der zur erhöhten Sicherheit der Überwachung der Infusionsfördermenge in Bezug auf den Sollwert zwei voneinander unabhängige IST-Werte erzeugt werden.

Diese Aufgabe wird erfindungsgemäss dadurch gelöst, dass zur Erzeugung zweier voneinander unabhängiger IST-Werte eine erste Fühlereinrichtung aus einem von der Pumpe angetriebenen Element und einer dieses abtastenden, an einen ersten Steuereingang des Rechnerbauteils angeschlossenen Abtastvorrichtung besteht und eine zweite Fühlereinrichtung durch einen in das Schlachsystem eingefügten Tropfensensor gebildet ist, der an einen zweiten, vom ersten getrennten Steuereingang des Rechnerbauteils angeschlossen ist, und dass die Alarmeinrichtung anspricht, wenn die Abweichung mindestens eines der IST-Werte vom SOLL-Wert einen vorgegebenen Wert erreicht.

So vorteilhaft der Einsatz eines elektronischen Rechnerbauteils bzw. eines Mikroprozessors ist, erhöht er auch die Anforderungen an dessen Überprüfung, um unkontrollierte Programmsprünge, Fehler im Programmspeicher, fehlerhafte Sprungadressen oder ein Hängenbleiben in einer Schleife zu vermeiden oder zu erkennen. Damit auch solche Fehler ausgeschaltet werden, sieht eine Ausgestaltung der erfindungsgemässen Vorrichtung vor, dass eine dritte Fühleinrichtung vorgesehen ist, die ein der Förderleistung entsprechendes unabhängiges IST-Signal erzeugt, das auf eine Auswerteschaltung gegeben wird, an die eine zweite Alarmeinrichtung angeschlossen ist, ein Signalgeber vorgesehen ist, der ein Überwachungssignal erzeugt, dessen Grösse abhängig ist von der in der Wähleinrichtung eingestellten Förderleistung und das in der Auswahlschaltung mit dem IST-Signal verglichen wird, und die Auswerteschaltung ein Alarmsignal erzeugt, wenn Überwachungssignal und IST-Signal ausserhalb einer vorgegebenen Relation sind. Mit Hilfe einer derartigen Überwachungsschleife wird das elektronische Rechnerbauteil überbrückt und unmittelbar festgestellt, ob die vorgewählte Fördermen-

ge innerhalb eines bestimmten Toleranzbereiches liegt. Ist dies nicht der Fall, wird Alarm gegeben. Parallel dazu kann auch der Antriebsmotor abgeschaltet werden.

Die dritte Fühleinrichtung kann wiederum beliebig sein, falls sie nur geeignet ist; vorteilhaft ist jedoch ein zweiter Tropfensensor.

Die in der Auswerteschaltung zu vergleichenden Signale können wiederum in geeigneter Art und Weise gebildet sein. Eine weitere Ausgestaltung der Erfindung sieht hierzu vor, dass das Überwachungssignal eine Impulsserie ist, bei der der Impulsabstand umgekehrt proportional zur eingestellten Förderleistung ist und die Auswerteschaltung die Anzahl der Impulse pro Tropfenabstand zählt. Hierbei wurde von folgender Überlegung ausgegangen: Die Impulsfrequenz des Überwachungssignals steigt mit steigender Förderleistung. Ferner ist das Zeitintervall zwischen zwei aufeinanderfolgenden Tropfen bei der geringsten Förderleistung am grössten und bei der höchsten Förderleistung am kleinsten. Dieses Intervall nimmt also proportional mit steigender Förderleistung ab. Hieraus folgt, dass die Anzahl der Impulse für den Zeitraum zwischen zwei Tropfen unabhängig ist von der Förderleistung und somit konstant. Die Auswerteschaltung zählt mithin die Anzahl der Impulse zwischen zwei Tropfen. Die Impulsanzahl muss einer vorgegebenen Konstanten (mit Toleranzabweichungen) entsprechen. Bei einer Abweichung liegt ein Fehler im Fördersystem vor. Die Auswerteschaltung löst einen Alarm aus und schaltet das Gerät ab.

Es ist bekannt, bei mit volumetrischen Pumpen arbeitenden Infusionsgeräten einen Satz von Pumpen einem einzigen Antriebsmotor zuzuordnen. Der Antriebsmotor, zum Beispiel ein Schrittschaltmotor, ist in einem Gehäuse angeordnet, das gleichzeitig Träger für die einzelnen Pumpen eines Pumpensatzes ist. Durch ein Aufsetzen einer Pumpe auf das entsprechend ausgebildete Gehäuse erfolgt automatisch eine Kupplung zwischen Antriebsmotor und Pumpe. Am Gehäuse sind ferner die Vorwähleinrichtungen für die Fördermenge und die Förderzeit angebracht. Wird nun etwa ein Mikroprozessor zur Steuerung oder Regelung eines derartigen Systems herangezogen, hat dies den Vorteil, dass durch eine entsprechende Eingabe in den Mikroprozessor eine entsprechende Umstellung auf die neu angekuppelte Pumpe erfolgen kann. Bekanntlich haben die Pumpen eines Pumpensatzes unterschiedliche Förderleistungen, was an sich auch erwünscht ist, um mit einem Gerät die Fördermenge zwischen weit auseinanderliegenden Werten zu variieren. In diesem Zusammenhang sieht eine weitere Ausgestaltung der Erfindung schliesslich vor, dass das Gehäuse und/oder der Antriebsmotor eine Erkennungseinrichtung enthält, die ein für jede Pumpe eines Satzes spezifisches Kennungssignal erzeugt, das auf einen weiteren Steuereingang des elektronischen Rechnerbauteils gegeben wird. Unabhängig von der jeweils verwendeten Pumpe kann mit einer einzigen Einstelleinrichtung die Fördermenge vorgewählt werden. Das Rechnerbauteil «erkennt» die jeweilige Förderleistung einer Pumpe und berücksichtigt dies durch Veränderung des Steuersignals für den Antriebsmotor, so dass stets für eine konstante Fördermenge pro Zeiteinheit gesorgt werden kann.

Ein Ausführungsbeispiel der Erfindung wird nachfolgend anhand von Zeichnungen näher beschrieben.

Fig. 1 zeigt ein Antriebsaggregat in Vorderansicht, teilweise geschnitten, ohne aufgesetzte Pumpe.

Fig. 2 zeigt eine Seitenansicht einer Schlauchpumpe.

Fig. 3 zeigt eine Draufsicht auf die Schlauchpumpe nach Fig. 2.

Fig. 4 zeigt eine Draufsicht auf das Antriebsaggregat nach Fig. 1.

Fig. 5 zeigt ein schematisches Schaltbild für eine Vorrichtung nach der Erfindung.

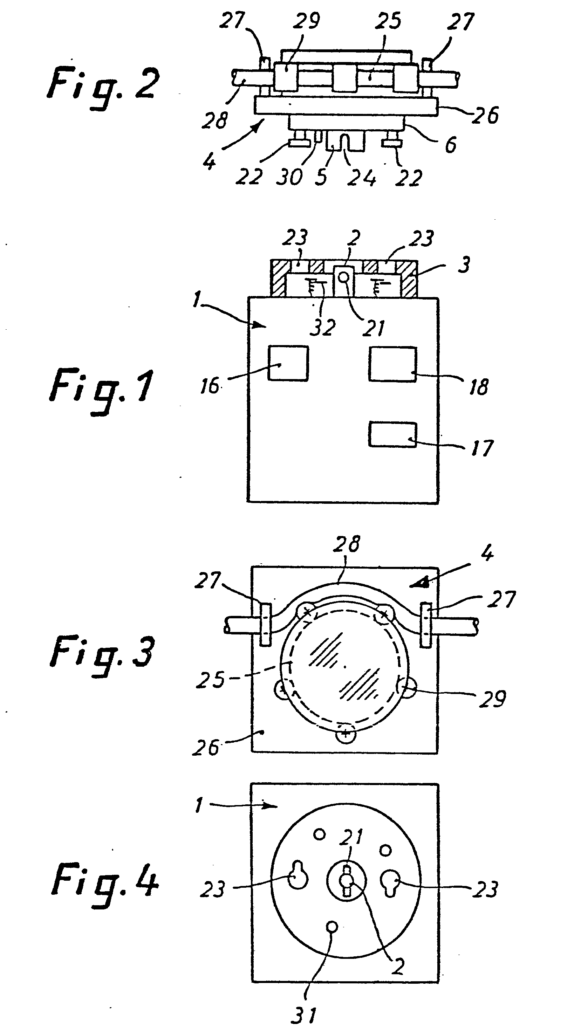

Das Antriebsaggregat 1 besitzt einen zum Antrieb einer Pumpe dienenden Wellenstumpf 2, der quer von einem Mitnehmerstift 21 durchsetzt ist. Der nach oben ragende Wellenstumpf 2 wird von einem am Aggregatgehäuse befestigten Kupplungsflansch 3 konzentrisch umgeben. Letzterer weist zwei Durchbrechungen 23 auf, die in der Flanschebene mit Verengungen versehen sind (Fig. 4). Der Kupplungsflansch 3 bildet von oben gesehen eine Lochplatte mit den Löchern 23. Das Antriebsaggregat 1 hat eine digitale Vorwähleinrichtung 16, die in ml/h skaliert ist und über einen nicht dargestellten Umsetzer bekannter Bauart die Drehzahl des Wellenstumpfes 2 und damit in später erläuterter Weise die Fördergeschwindigkeit der Flüssigkeit durch eine Pumpe bestimmt. Das Antriebsaggregat 1 weist weiterhin einen digitalen Summenzähler 17 auf, der in ml skaliert ist und zur Erfassung und Anzeige der bereits geförderten Flüssigkeitsmenge dient. Das Aggregat 1 ist weiterhin mit einer digitalen Vorwähleinrichtung 18 versehen, mittels der die gesamte zu fördernde Flüssigkeitsmenge eingestellt wird. Wenn diese Gesamtmenge erreicht worden ist, was dann auch am Summenzähler 17 angezeigt wird, wird das Flüssigkeitsfördergerät so abgeschaltet, dass der Wellenstumpf 2 stillsteht.

Die in den Fig. 2 und 3 gezeigte Schlauchpumpe 4 hat eine feststehende Montageplatte 6, die mit Hammerköpfen versehene Stecker 22 trägt. Diese Platte 6 ist an den Flansch 3 des Aggregates 1 anlegbar, wobei die Stecker 22 durch die Löcher 23 des Flansches ragen, so dass die Hammerköpfe dieser Stecker die Dicke des Flansches 3 überragen, so dass dann die Platte 6 mit Stecker 22 gegenüber der Platte 3 etwas verdreht werden kann, so dass die Hammerköpfe den Flansch 3 hintergreifen. Die Schlauchpumpe hat weiterhin eine Platte 26, in der die Schlauchabpressscheibe 25 drehbar gelagert ist. An der Platte 26 sitzen Schlauchhalterungen 27 für einen in die Schlauchpumpe eingelegten Schlauch 28. Die Scheibe 25 ist auf der drehbaren Antriebswelle 5 befestigt. Die Scheibe 25 ist mit mehreren über den Umfang verteilt angeordneten Pressrollen 29 versehen. Beim Drehen der Scheibe 25 mit den Rollen 29 drücken die Rollen den Schlauch 28 zusammen und bewegen sich längs diesem Schlauch, so dass die im Schlauch 28 befindliche Flüssigkeit gefördert wird. Die Schlauchpumpe 4 wird auf das Aggregat 1 aufgesetzt und durch geringes seitliches Verdrehen daran fixiert. Hierbei ist der Wellenzapfen 2 ins Innere der Hohlwelle 5 eingetreten und der Mitnehmerstift 21 liegt in der Querrinne 24 der Welle 5. Auf diese Weise ist eine drehfeste Kupplung zwischen Aggregat 1 und Pumpe 4 entstanden. Auf die gleiche einfache Weise ist die Pumpe wieder vom Aggregat 1 lösbar. Die jeweilige Drehzahl des Wellenstumpfes 2 bestimmt die Fördergeschwindigkeit der Flüssigkeit im Schlauch 28 und damit die Fördermenge in der Zeiteinheit.

Anstelle der Schlauchpumpe nach den Fig. 2 und 3 kann beispielsweise auch eine Kolbenpumpe oder eine Flügelzellenpumpe oder ein Einmalgerät anderer Art, wie Faltenbalg, eingesetzt werden, die jedoch einen identischen Unterbau besitzt, damit sie mit dem Kupplungsflansch 3 in Eingriff gebracht werden kann.

Für die jeweilige Pumpenart ist jedoch ein Kontaktstift 30 typisch, der federnd in einer Öffnung der Montageplatte 6 sitzt und zur Gänze in die Öffnung der Montageplatte eingedrückt werden kann. Entsprechend weist der Montageflansch 3 an der Oberseite drei Öffnungen 31 auf, unterhalb denen Kontaktpaare 32 (siehe Fig. 1) angeordnet sind. Von einem Antriebsaggregat wird jeweils nur ein Kontaktpaar betätigt, so dass das Antriebsaggregat feststellen kann, welche Pumpe gerade im Einsatz ist. Hierauf wird jedoch noch weiter unten eingegangen.

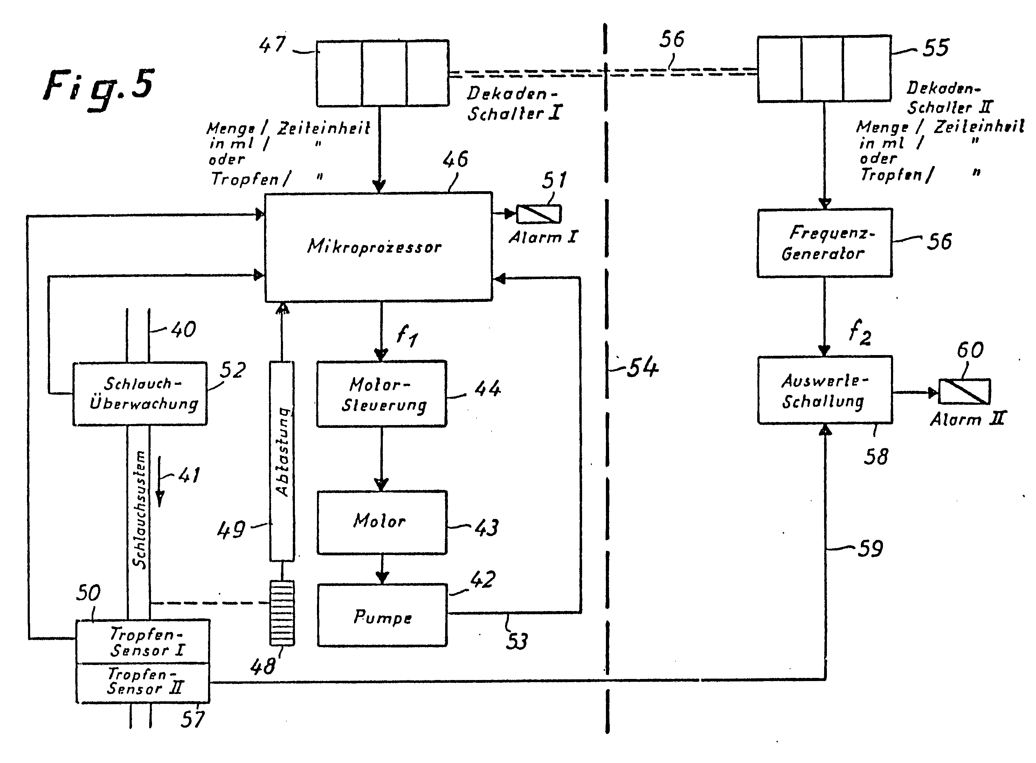

Fig. 5 zeigt die einzelnen Blöcke einer Infusionsvorrichtung, welche durch Wirkungslinien miteinander verbunden sind. Das schematisch dargestellte Schlauchsystem zwischen Vorrat an Infusionslösung und Infusionskanüle ist allgemein mit 40 bezeichnet, und die Fliessrichtung ist mit dem Pfeil 41 angedeutet. Mit dem Schlauchsystem 40 wirkt, etwa wie in den Fig. 1 bis 4 dargestellt, eine Schlauchpumpe 42 zusammen, um Infusionslösung durch das Schlauchsystem 40 zu fördern. Die Infusionspumpe 42 wird mit Hilfe eines Motors 43 angetrieben, z.B. einem elektrischen Schrittmotor. Der Steuerblock für den Motor 43 ist mit 44 bezeichnet.

Für die Steuerung und Überwachung aller Funktionen der Vorrichtung nach Fig. 5 ist ein Mikroprozessor 46 vorgesehen. Die Ansteuerung des Mikroprozessors mit einem Signal für die gewählte Fördermenge pro Zeiteinheit, zum Beispiel in ml oder in Tropfen pro Zeiteinheit erfolgt über einen sogenannten Dekadenschalter 47. Wird mittels des Dekadenschalters 47 eine vorgegebene Menge pro Zeiteinheit eingestellt, rechnet daraus der Mikroprozessor das entsprechende Steuersignal für die Motorsteuerung aus, beispielsweise eine bestimmte Impulsserie mit vorgegebener Impulsfrequenz.

Mit der Pumpe 42 ist eine Rasterscheibe 48 gekoppelt, die mit Hilfe einer Abtastvorrichtung 49 abgetastet ist. Die Abtastvorrichtung 49 erzeugt ein Signal, dessen Grösse abhängig ist von der Drehzahl der Rasterscheibe 48. Dieses Signal wird auf einen Steuereingang des Mikroprozessors 46 gegeben. Mit Hilfe der Rasterscheibe 48 und der Abtastvorrichtung 49 kann somit ein IST-Signal gebildet werden, das im Mikroprozessor mit dem Einstellsignal für die Förderleistung verglichen wird. Eine Regelabweichung führt zu einer entsprechenden Veränderung der Motordrehzahl.

Im Schlauchsystem ist ferner ein Tropfensensor 50 angeordnet, der aus der Anzahl der Tropfen pro Zeiteinheit ein Signal erzeugt, das auf einen anderen Steuereingang des Mikroprozessors gegeben wird. Auch hierdurch lässt sich somit ein IST-Signal bilden im Hinblick auf die tatsächliche Förderleistung, das im Mikroprozessor mit der voreingestellten Tropfenzahl pro Zeiteinheit verglichen werden kann zwecks Bildung einer Regelabweichung. Normalerweise wird nur eine Regelungsschleife erforderlich sein, entweder über den Tropfensensor oder über die Abtastung der Rasterscheibe; es ist jedoch möglich, beide IST-Signale gleichwohl in den Mikroprozessor einzugeben und ein Alarmsignal zu erzeugen, wenn eines der beiden IST-Signale eine vorbestimmte Abweichung vom Sollwert aufweist. Der Mikroprozessor erzeugt dann ein Alarmsignal, das auf eine optische und akustische Alarmeinrichtung 51 gegeben wird.

Im Schlauchsystem 40 ist ausserdem eine Schlauchüberwachung 52 angeordnet, die ermittelt, ob überhaupt Flüssigkeit gefördert wird oder ob Luft im Schlauchsystem ist. Eine derartige Überwachung kann zum Beispiel mit Hilfe einer elektrooptischen Vorrichtung durchgeführt werden, welche jedoch Stand der Technik ist.

Motor 43 und Pumpe 42 können entsprechend der Ausführungsform nach den Fig. 1 und 4 ausgebildet sein, d.h. einem Motor 43 ist ein Pumpensatz zugeordnet. Durch die Zuordnung einer bestimmten Pumpe eines Satzes zum Motor wird über eine Wirkungslinie 53 ein Kennungssignal auf den Mikroprozessor gegeben, der somit «weiss», welche Pumpe gerade im Einsatz ist. Der Mikroprozessor berücksichtigt dies bei der Abgabe des Steuersignals f1, so dass die Förderleistung der Motor-Pumpeneinheit immer nur dem eingestellten Leistungswert entspricht, unabhängig davon, welche Pumpe eines Pumpensatzes im Einsatz ist. Wie im Zusammenhang mit der Ausführungsform nach den Fig. 1 bis 4 erläutert, erfolgt die Erkennung der jeweils verwendeten Pumpe automatisch. Entsprechend ist die automatische Berücksichtigung bei der Steuerung des Motors zum Antrieb der Pumpe.

Da, wie erwähnt, alle Funktionen zum Betrieb der Förderpumpe 42 über den Mikroprozessor 46 laufen und es immerhin geschehen kann, dass in diesem ein Fehler eingebaut ist, gehört zur Vorrichtung nach Fig. 5 eine zusätzliche Überwachungsvorrichtung, welche auf der rechten Seite der gestrichelten Linie 54 angeordnet ist. Sie enthält einen zweiten Dekadenschalter 55, der mit dem Dekadenschalter 47 integriert ist und von letzterem über die Wirkungslinie 56 entsprechend dem Dekadenschalter 47 auf die vorgewählte Förderleistung in Fördermenge pro Zeiteinheit oder Tropfen pro Zeiteinheit eingestellt wird. Der Dekadenschalter 55 erzeugt ein dieser voreingestellten Förderleistung entsprechendes Signal, das auf einen Frequenzgenerator 56 gegeben wird, der seinerseits eine Impulsfolge erzeugt, wobei die Impulsfrequenz proportional ist der eingestellten Förderleistung. Ein zweiter Tropfensensor 57 zählt die Zahl der Tropfen im Schlauchsystem 40 und gibt z.B. für jeden Tropfen einen Impuls auf eine Auswerteschaltung 58 über die Wirkungslinie 59. Die Auswerteschaltung 58 erhält auch die Impulsfolge des Frequenzgenerators 56. In der Auswerteschaltung wird nun ermittelt, wieviele Impulse der Impulsfolge des Frequenzgenerators 56 auf den Abstand zwischen zwei Tropfen kommen. Durch die Proportionalität der Impulsfrequenz zur Förderleistung muss die Anzahl der Impulse des Frequenzgenerators 56 im Intervall zwischen zwei Tropfen immer gleich sein, unabhängig von der jeweiligen Förderleistung. Weicht die Anzahl der Impulse f2 erheblich von dem vorgegebenen Wert ab (unter Berücksichtigung von Toleranzen), liegt ein Fehler im System vor, so dass die Auswerteschaltung 58 ein Alarmsignal erzeugt, das auf eine zweite Alarmeinrichtung 60 gegeben wird. Sowohl die Erzeugung eines Alarmsignals vom Mikroprozessor 46 für die Alarmeinrichtung 51 als auch die Erzeugung des Alarmsignals von der Auswerteschaltung 58 für die zweite Alarmeinrichtung 60 führt zu einer sofortigen Abschaltung des Antriebsmotors 43 für die Pumpe 42, so dass eine Beeinträchtigung eines Patienten durch einen Fehler in der Infusionsvorrichtung vermieden wird.

Die in Fig. 5 dargestellte Anordnung kann auch für eine sogenannte externe Ansteuerung verwendet werden, z.B. durch einen Rechner, der mit zusätzlichen Diagnosewerten gespeist wird. Für diesen Fall kann zum Beispiel der Tropfensensor 57 ein IST-Signal für den externen Rechner bilden, so dass im Rechner ein SOLL-IST-Wert-Vergleich vorgenommen wird, d.h. ein Vergleich des IST-Signals mit dem Dosiersollwert.

Zur Feststellung der Betriebssicherheit vor Inbetriebnahme der Pumpe 42 kann der Anforderungsanfall (Grossabweichung von SOLL- und IST-Wert) simuliert werden, um so die Fehlerfreiheit beider Kanäle und der Alarmeinrichtungen 51, 56 zu überprüfen.

## Patentansprüche

1. Vorrichtung zum Infundieren und Injizieren von Flüssigkeiten, mit einer Pumpe (42) in einem Schlauchsystem zwischen einem Vorrat an Infusionslösung und einer Infusionskanüle oder dergleichen, einem in seiner Drehzahl steuerbaren Antriebsmotor (43) für die Pumpe (42), einer ein elektronisches Rechnerbauteil (46) enthaltenden Steuereinrichtung für den Motor, die eine Wähleinrichtung (47) für den SOLL-Wert der von der Pumpe zu fördernden Flüssigkeitsmenge umfasst, mit einer Fühlereinrichtung, die die Förderleistung der Pumpe misst und ein IST-Signal über einen Steuereingang auf das elektronische Rechnerbauteil gibt, das abhängig von einem SOLL-IST-Wertvergleich das Steuersignal für den Motor (43) bildet, sowie mit einer an das Rechnerbauteil angeschlossenen Alarmeinrichtung (51) für einen vorgegebenen Grenzwert des SOLL-IST-Wertvergleichs, dadurch gekennzeichnet, dass zur Erzeugung zweier voneinander unabhängiger IST-Werte eine erste Fühlereinrichtung aus einem von der Pumpe (42) angetriebenen Element (48) und einer dieses abtastenden, an einen ersten Steuereingang des Rechnerbauteils (46) angeschlossenen Abtastvorrichtung (49) besteht und eine zweite Fühlereinrichtung durch einen in das Schlauchsystem (40) eingefügten Tropfensensor (50) gebildet ist, der an einen zweiten, vom ersten getrennten Steuereingang des Rechnerbauteils (46) angeschlossen ist, und dass die Alarmeinrichtung (51) anspricht, wenn die Abweichung mindestens eines der IST-Werte vom SOLL-Wert einen vorgegebenen Wert erreicht.

2. Vorrichtung nach Anspruch 1, dadurch gekennzeichnet, dass zur Überwachung des Rechnerbauteils (46) zusätzlich eine dritte Fühlereinrichtung (57) vorgesehen ist, die ein der Förderleistung entsprechendes unabhängiges IST-Signal erzeugt, das auf eine Auswerteschaltung (58) gegeben wird, an die eine Alarmeinrichtung (60) angeschlossen ist, ein Signalgeber (56) vorgesehen ist, der ein Überwachungssignal erzeugt, dessen Grösse abhängig ist von der in der Wähleinrichtung (47, 55) eingestellten Förderleistung und das in der Auswerteschaltung (58) mit dem IST-Signal verglichen wird, so dass die Auswerteschaltung (58) ein Alarmsignal erzeugt, wenn Überwachungssignal und IST-Signal ausserhalb einer vorgegebenen Relation sind.

3. Vorrichtung nach Anspruch 2, dadurch gekennzeichnet, dass die dritte Fühleinrichtung ein weiterer in das Schlauchsystem (40) eingefügter Tropfensensor (57) ist.

4. Vorrichtung nach Ansprüchen 2 und 3, dadurch gekennzeichnet, dass das Überwachungssignal eine Impulsfolge ist, bei der der Impulsabstand umgekehrt proportional zur eingestellten Förderleistung ist und die Auswerteschaltung (58) die Anzahl der Impulse pro Tropfenabstand zählt und mit einem vorgegebenen Wert vergleicht.

5. Vorrichtung nach einem der Ansprüche 1 bis 4, bei der ein Satz Pumpen einem Antriebsmotor zugeordnet ist, der in einem Gehäuse angeordnet ist, das gleichzeitig als Träger für eine Pumpe dient, dadurch gekennzeichnet, dass das Gehäuse (1) und/oder der Antriebsmotor (43) eine Erkennungseinrichtung (31, 32, 53) enthält, die für jede Pumpe eines Satzes ein spezifisches Kennungssignal erzeugt, das auf einen weiteren Steuereingang des elektronischen Rechnerbauteils (46) gegeben wird.

## Claims

1. An apparatus for infusing and injecting liquids, comprising a pump (42) in a hose system between a supply of infusion solution and an infusion cannula or the like, a speed controllable driving motor (43) for the pump (42), a control means for the motor comprising an electronic computer structural member (46), said control means comprising a selector means (47) for the preset value of the amount of liquid to be discharged by the pump, with a sensing means measuring the discharge output of the pump and providing an actual value signal to the electronic computer structural member via a control input, said computer structural member forming the control signal for the motor (43) in dependence upon a preset value/actual value comparison, as well as with an alarm means (51) connected to the computer structural member for a predetermined limit value of the preset value/actual value comparison, characterized in that for the generation of two actual values independent of each other a first sensor means consists of an element (48) driven by the pump (42) and a scanning means (49) connected to a first control input of the computer structural member (46) and scanning the element (48), and a second sensor means is formed by a drop sensor (50) inserted into the hose system (40) and connected to a second control input of the computer structural member (46) separated from the first control input, and in that the alarm means (51) becomes responsive when the deviation of at least one of the actual values from the preset value reaches a predetermined value.

2. An apparatus according to claim 1, characterized in that for the supervision of the computer structural member (46) a third sensor means (57) is additionally provided which generates an independent actual value signal corresponding to the discharge output and which is transmitted onto an evaluation circuit (58) having an alarm means (60) connected thereto with a signal generator (56) provided which generates a control signal the quantity of which is dependent on the discharge output adjusted in the selector means (47, 55) and which is compared to the actual value signal in the evaluation circuit (58) so that the evaluation circuit (58) generates an alarm signal when the control signal and the actual value signal are outside a predetermined relationship.

3. An apparatus according to claim 2, characterized in that the third sensor means comprises another drop sensor (57) inserted into the hose system (40).

4. An apparatus according to claims 2 and 3, characterized in that the control signal comprises a succession of pulses wherein the space between the pulses is inversely proportional to the adjusted discharge output and the evaluation circuit (58) counts the number of pulses per drop interval and compares it to a predetermined value.

5. An apparatus according to any one of the claims 1 to 4, with a set of pumps associated to a driving motor, said driving motor arranged in a housing which at the same time serves as a carrier for a pump, characterized in that the housing (1) and/or the driving motor (43) contains a recognition device (31, 32, 53) which generates a specific recognition signal for each pump in a set which signal is transmitted to a further control input of the electronic computer structural member (46).

**Revendications**

1. Dispositif pour infuser et injecter des liquides, comportant une pompe (42), dans un système de tuyau flexible, entre un réservoir à solution d'infusion et une canule d'infusion, ou similaire, un moteur d'entraînement (43), à vitesse réglable, pour la pompe (42), un moyen de commande du moteur contenant un élément de calcul électronique (46) qui englobe un moyen de sélection (47) de la valeur de consigne de la quantité de liquide à fournir par la pompe, un capteur qui mesure le débit de la pompe et délivre, en passant par une entrée de commande, un signal de valeur réelle à l'élément de calcul électronique, qui, en fonction de la comparaison entre les valeurs de consigne et réelle, génère le signal de commande du moteur (43), ainsi qu'un moyen d'alarme (51), raccordé à l'élément de calcul, pour une valeur limite prédéterminée de la comparaison entre les valeurs de consigne et réelle, caractérisé par le fait que, pour obtenir deux valeurs réelles indépendantes l'une de l'autre, un premier capteur est constitué par un élément (48) entraîné par la pompe (42) et un dispositif d'échantillonnage (49) explorant cet élément (48) et raccordé à une première entrée de commande de l'élément de calcul, et un second capteur est formé par un détecteur de gouttes (50) inserré dans le système de tuyau (40), qui est raccordé à une seconde entrée de commande de l'élément de calcul (46) séparée de la première, et le moyen d'alarme (51) répond lorsqu'au moins une des valeurs réelles s'écarte de la valeur nominale d'une quantité déterminée.

2. Dispositif selon la revendication 1, caractérisé par le fait que, pour surveiller l'élément de calcul (46), il est prévu en outre un troisième capteur (57) qui génère un signal de valeur réelle, indépendant, correspondant au débit, qui est transmis à un circuit d'exploitation (58) auquel est raccordé un moyen d'alarme (60); il est prévu un générateur de signal (56) qui produit un signal de surveillance dont la grandeur dépend du débit réglé dans le moyen de sélection (47, 55) et qui est comparé, dans le circuit d'exploitation (58), avec le signal de valeur réelle, de sorte que le circuit d'exploitation (58) produit un signal d'alarme lorsque le signal de surveillance et le signal de valeur réelle ne sont pas dans une relation prédéterminée.

3. Dispositif selon la revendication 2, caractérisé par le fait que le troisième capteur est un autre détecteur de gouttes (57) introduit dans le système de tuyau (40).

4. Dispositif selon la revendication 2 ou 3, caractérisé par le fait que le signal de surveillance est un train d'impulsions dans lequel l'intervalle d'impulsion est inversement proportionnel au débit réglé et le circuit d'exploitation (58) compte le nombre des impulsions par intervalle entre gouttes et le compare à une valeur prédéterminée.

5. Dispositif selon l'une des revendications 1 à 4, dans lequel un jeu de pompes est associé à un moteur de commande qui est disposé dans un boîtier servant également de support d'une pompe, caractérisé par le fait que le boîtier (1) et/ou le moteur de commande (43) contient un moyen de reconnaissance (31, 32, 53) qui fournit, pour chaque pompe d'un jeu, un signal de reconnaissance spécifique, qui est transmis à une autre entrée de commande de l'élément de calcul électronique (46).

0 028 364

Fig. 2

Fig. 1

Fig. 3

Fig. 4

9

Fig.5

0 028 364